# EUROPEAN PATENT SPECIFICATION

(11) **EP 1 515 759 B1**
(45) Date of publication and mention of the grant of the patent: **05.10.2005**
(21) Application number: 03740162.7
(22) Date of filing: 30.05.2003
(51) Int. Cl.: A61L 27/30, C25D 9/06

(54) **AN OSTEOINTEGRATIVE INTERFACE FOR IMPLANTABLE PROSTHESES AND METHOD FOR ITS MANUFACTURE**
OSTEOINTEGRIERENDE GRENZFLÄCHE FÜR IMPLANTIERBARE PROTHESE UND METHODE ZU DEREN HERSTELLUNG
INTERFACE D'OSTEOINTEGRATION POUR PROTHESES IMPLANTABLES ET PROCEDE DE FABRICATION

(30) Priority: 21.06.2002 IT MI20021377
(43) Date of publication of application: 23.03.2005
(73) Proprietor: Politecnico Di Milano, 20133 Milano (IT)
(72) Inventor: CIGADA, Alberto, I-20123 Milano (IT); CHIESA, Roberto, I-21040 Vedano Olona (IT); SANDRINI, Enrico, I-25122 Brescia (IT); RONDELLI, Gianni, I-20100 Milano (IT); SANTIN, Matteo, Brighton (GB)
(74) Representative: Cattaneo, Elisabetta
(86) International application number: PCT/EP2003/005686
(87) International publication number: WO 2004/000378

(56) References cited:
- WO-A-02/096475
- US-A- 4 801 300
- US-A- 5 385 662
- US-A- 5 478 237
- US-A- 5 723 038
- KOKUBO T ET AL: "Ceramic-metal and ceramic-polymer composites prepared by a biomimetic process" COMPOSITES PART A: APPLIED SCIENCE AND MANUFACTURING, ELSEVIER SCIENCE PUBLISHERS B.V., AMSTERDAM, NL, vol. 30, no. 4, April 1999 (1999-04), pages 405-409, XP004158190 ISSN: 1359-835X

## Description

The present invention relates to an osteointegrative interface for implantable prostheses and to a method for the treatment of the osteointegrative interface, particularly to a biomimetic osteointegrative interface and to a method for modifying the surface of the osteointegrative interface superficially.

Biomimetic processes which simulate biomineralization processes have been used with success for preparing materials such as iron oxides, apatite, and cadmium sulphide which are used in various technological fields.

The field which has drawn most "inspiration" from biomineralization processes is the field of biomaterials and, in particular, biomaterials for orthopaedics, maxillo-facial surgery, and dentistry.

These materials have to interact with body tissues and should therefore possess particular biocompatibility and bioactivity qualities and mechanical properties.

The main uses of biomaterials in the hard tissues field include prostheses such as hip prostheses, knee prostheses, dental implants, screws, nails, plates and osteosynthesis means.

Because of the mechanical properties required, the materials usually used for prostheses are stainless steel, titanium, titanium alloys, and tantalum, which have the best biocompatibility characteristics of all of the metals.

These metals exhibit a high mechanical breaking load but require long implantation periods in order for their integration with the biological tissues such as, for example, bone, to be established.

To prevent this problem, there are known biomimetic treatments for osteointegrative interfaces, by means of which it is possible to bring about a significant increase in the rate of precipitation of hydroxyapatite after immersion in biological fluids, and its adhesion to the prosthesis.

Calcium phosphates, including hydroxyapatite, are the main constituents of the inert bone matrix.

US patents 5,152,993 (Lars Magnus *et al*.) and 5,885,612 (Ohthuke *et al*.) propose a treatment in hydrogen peroxide with the use of hydrogen peroxide or hydrogen peroxide and metal ions, which modifies the surface of the prosthesis, promoting the formation of -OH hydroxyl functional groups which can induce the formation of a stable interface with bone.

US patent 5,609,633 (Kokubo) and the corresponding application EP 678300A1 (Kokubo) teach a treatment based on immersion in an alkaline solution such as NaOH, KOH or CaOH₂, followed by washing and high-temperature heat treatment.

In particular, these two documents describe a treatment for etching with hydroxides an oxide such as titanium oxide, which is almost completely crystalline and is composed mainly of the rutile, anastase and, rarely, brookite phases, which are different crystalline forms of titanium dioxide, that is TiO₂.

This etching has the advantage not only of forming a larger number of -OH groups so that the surface layer hydroxylates more easily and is particularly significantly enriched with oxygen, but also of achieving the formation of a layer of amorphous calcium phosphates which are stoichiometrically similar to hydroxyapatite and can therefore promote the formation of mature hydroxyapatite.

The heat treatment included in this treatment with alkalis would lead to an increase of up to about 1 µm in the thickness of the titanium oxide layer.

To achieve a significant increase in the thickness of oxide which forms on the surface of a metal, anodic passivation treatments have been optimized by the development of a technique known as anodic spark deposition (ASD).

The ASD technique is a galvanic electrodeposition process which is performed at fairly high voltage and which causes point breakdown and perforation of the dielectric surface oxide layer which is formed progressively, thus allowing it to grow.

With this technique, as described, for example, in US patent 5,385,662 (Kurze *et al*.), it is possible to produce coatings with the characteristics of sintered ceramic materials, which are particularly resistant to abrasion and corrosion, and with thicknesses of up to 150 µm.

with specific reference to bone implants, US patent 5,478,237 (Ishizawa) teaches the use of this technique to bring about a modification of the composition, of the thickness, and of the morphology of the titanium oxide film, that is, the formation of a thick and nanoporous oxide film containing calcium and phosphorus, of a thickness clearly greater than that which is formed by natural oxidation of the metal.

However, this technique does not substantially improve the biomimetic properties of the titanium.

In order to improve its biomimetic capacity, the film or layer is subjected to a hydrothermic treatment which can promote nucleation of hydroxyapatite crystals. However, these crystals are not distributed homogeneously and do not cover the surface of the osteointegrative interface completely.

Homogeneity of behaviour is therefore not achieved.

Moreover, the mechanical bond between oxide and crystals is very weak.

US 5 723 038 describes a process for producing a gradient coating of calcium phosphate phases and metal oxide phases on metallic implants to accelerate in growth of cells.

In view of the prior art described, the object of the present invention is to modify superficially the surface of titanium, of tantalum, and of their alloys so as to produce biomimetic surfaces having structural and morphological characteristics that are innovative in comparison with those known from the prior art.

According to the present invention, this object is achieved by a biomimetic osteointegrative interface according to Claim 1.

This object is also achieved by a method for the production of a biomimetic osteointegrative interface according to Claim 4.

By virtue of the present invention, it is possible to achieve an increase in the thickness of the oxide film of titanium, of tantalum, or of their alloys from a few nanometres to a few micrometres by reducing, amongst other things, the extent to which metal ions are released from the implant. The adhesion of the oxide layer to the substrate is excellent.

The present invention, also enables elements such as calcium and phosphorus which can promote mineralization processes of the extracellular bone matrix to be incorporated in the oxide film of titanium, of tantalum, or of their alloys.

Moreover, by virtue of the present invention it is possible to bring about, for example, in a sponge, the formation of nanometric porosity which can geometrically promote protein adhesion and consequently the adhesion of osteoblast cells.

Finally, by virtue of the present invention, it is possible to bring about the creation, within the titanium oxide film, that is, in its porosity, of -OH bonds which can form chemical bonds both with calcium and phosphorus ions, further promoting the mineralization of the extracellular matrix, and with proteins, further promoting osteoblast adhesion.

The characteristics and the advantages of the present invention will become clear from the following detailed description of a practical embodiment thereof which is illustrated by way of non-limiting example in the appended drawing, in which:
Figure 1 shows, in section, an embodiment of an osteointegrative interface according to the present invention, and
Figure 2 is a section, taken on the line II-II, of the layers making up the surface of the osteointegrative interface of Figure 1.

One of the most important requirements in applications for osteointegrative interfaces is to synthesize a surface with crystals of dimensions comparable to those of biological apatite, so that an increase in the surface area of the crystals can improve the interactions at the interface with the bone tissue and increase the capability of the material thus to create a bond with the bone tissue.

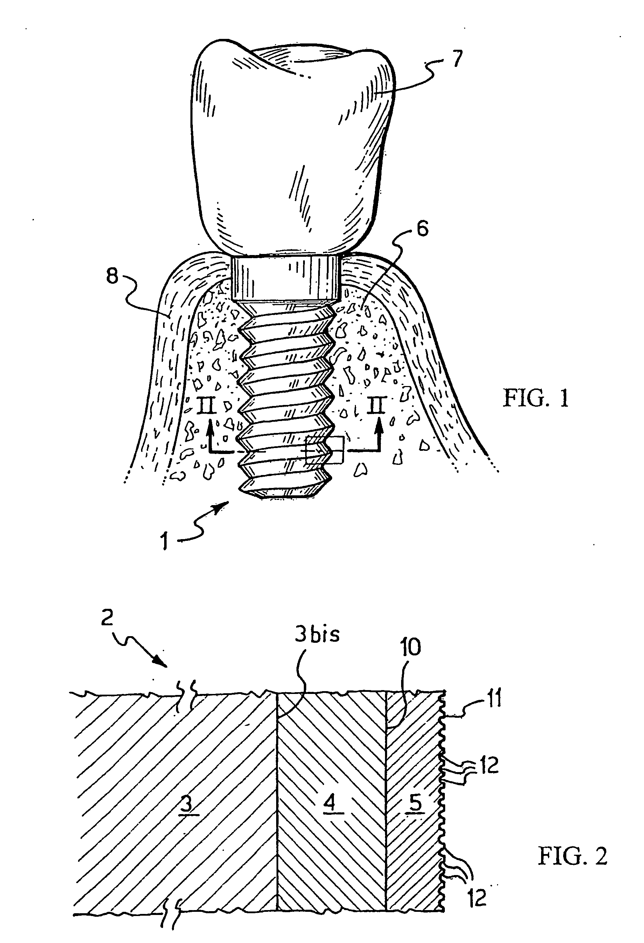

With reference to appended Figures 1 and 2, which show an example of an osteointegrative interface for a dental implant 1 according to the present invention, this requirement is satisfied.

with reference in particular to Figure 2, an enlarged section through the implant 1, taken on the line II-II of Figure 1 so as to show the layers making up the surface of the osteointegrative interface 1, is indicated 2.

These layers comprise a substrate 3 generically of titanium, a protective layer 4, generically of titanium oxide, superimposed on the surface 3bis of the substrate 3, and a surface layer 5 superimposed on the protective layer 4.

Figure 1 shows the dental implant 1 which has, for example, the shape of a screw, and which has been inserted in a bone socket 6; a tooth crown is indicated 7 and the gum is indicated 8.

Naturally, the shape and size of the implant must be selected in dependence on the specific application and the example shown in Figure 1 is only one of the possible embodiments.

In particular, the substrate 3 which constitutes the core of the dental implant 1 is composed of transition metals, for example, of titanium, of tantalum, or of their alloys.

If the substrate 3 is made of titanium, the protective layer 4 will comprise titanium oxide which is formed by the process described below to reach a thickness of the order of a few µm, for example 5 - 10 µm.

In the innovative embodiment, this protective layer 4 is enriched throughout its thickness of 5 - 10 µm with a relatively uniform and high concentration of elements such as calcium and phosphorus, the concentration of the element calcium being greater than the concentration of phosphorus, more than 2 times greater, that is Ca/P > 2.

The surface layer 5, which has a thickness of the order of tens of nanometres and is superimposed on the protective layer 4, comprises the same elements as the protective layer 4 but these elements have concentration ratios between calcium and titanium, that is Ca/Ti, and between phosphorus and titanium, that is P/Ti, which tend to be greatly in favour of the elements which constitute the enrichment, that is (Ca + P)/Ti > 80%. Moreover the Ca/P ratio is higher and is equal to about Ca/P ≅ 3 or even more.

Moreover, it can be seen that the surface layer 5 has a lower surface 10 and an upper surface 11, the lower surface 10 being in contact with the titanium oxide layer 4 and the upper surface 11 being in contact with the bone socket 6.

The layer 5, particularly on the upper surface 11, is also characterized by the presence of a high concentration of -OH chemical coupling groups, suitable for forming a plurality of nucleation centres 12.

The osteointegrative interface thus described is produced by a series of steps which are described below.

In a first step, the surface 3bis of the implant 1 is subjected to a mechanical or even chemical finishing treatment with abrasive paper, sandblasting, or the like, to produce a surface with controlled and homogeneous roughness with Ra values of the order of 1-2 µm for dental implants and greater values for non-cemented orthopaedic prostheses.

There is then a second step of cleaning of the upper surface 3bis with the use, for this purpose, of an ultrasound chamber containing acetone for a first period of time included within a time interval where 3 < t < 5 minutes and distilled water for a second period of time, where 3 < t < 5 minutes.

This step is useful since it enables dirt particles and/or impurities to be removed from the surface 3bis of the substrate 3.

A third step is then provided for; in this step a first anodic spark deposition (ASD) treatment takes place in an aqueous calcium glycerophosphate (Ca-GP) solution at a concentration of 0.015 M with a maximum variation of about ± 0.005M. This step provides for treatment at about T = 0°C, preferably with a maximum variation of ± 1°C and with a predetermined current-intensity value of about 70 A/m², whilst the potential rises freely to a predetermined final value of about 350 V. This brings about the growth of the protective layer 4 and the deposition therein of a predetermined quantity of phosphorus and also some calcium.

The mechanical adhesion of the protective layer to the substrate is outstanding.

The third step ends when the potential reaches 350 V and the current intensity is still about 70 A/m².

A fourth step is then performed in which washing in distilled water and careful drying of the osteointegrative implant 1 thus treated take place.

The fifth step provides for a second ASD treatment in an aqueous solution of calcium hydroxide [Ca(OH)₂] at a concentration of 0.1 M with a maximum variation of ± 0.02M. For a first period of time, the treatment is performed at a temperature of between two and eight degrees centigrade, that is 2°C < T < 8°C, with a constant current intensity of about 70 A/m² whilst the potential is allowed to rise to a final value of about 370 V.

The fifth step continues for a second period of time with deposition at a constant voltage of about 370 V and simultaneous reduction in current to a predetermined value of about 35 A/m².

The layer 4 is thus modified and incorporates further calcium.

It should be noted that the final current intensity is equal to half of the initial current used during the first stage of the second ASD treatment.

There is then a sixth step which provides for further washing in distilled water and careful drying of the implant 1 thus treated.

The seventh step provides for the immersion of the osteointegrative implant 1 thus treated in 5 M aqueous KOH solution (or even NaOH, but with less satisfactory results) kept at T = 60°C for t = 24 h.

Finally, an eighth step is provided for; in this step a final washing in distilled water and careful drying of the interface thus treated take place.

In particular, it should be noted that the third step, that is the first ASD, produces a titanium oxide layer 4 which is rich in phosphorus and partially in calcium so as to give rise to a dielectric with a thickness of the order of a few µm (possibly about ten µm) which permits the application of the high voltages that are required in the second ASD.

In fact, without a first deposition and thickening of the surface oxide, it is impossible to bring the osteointegrative implant 1 to a voltage high enough to perform the second ASD treatment in Ca(OH)₂ solution, which is a solution without anions such as to be able to ensure the formation of an adequate dielectric thickness.

Basically, whereas with the first ASD a titanium oxide layer of the desired thickness, provided with a first concentration of calcium and phosphorus, is formed, with the second ASD treatment, the layer is thickened and there is an increase in the Ca/P ratio.

Moreover, the second ASD treatment is preparatory to the formation of nucleation centres characterized by the presence of surface -OH chemical groups.

The seventh step, that is, the immersion of the interface 1 in a KOH solution, further increases the Ca/P ratio, since phosphorus is extracted from the surface portion of the layer 4 so as to favour its hydration and a high concentration of -OH groups is produced on the surface.

The fifth step and the seventh step thus produce a surface layer 5 in which the Ca/Ti and P/Ti ratios tend to be greatly in favour of the two elements which constitute the enrichment, to the extent that the surface layer 5 is composed, in its last nanometres, substantially by calcium and phosphorus, with a Ca/P ratio close to four and in any case no less than three.

Moreover, by virtue of the innovative treatment method, the surface layer 5 is rich in -OH chemical coupling groups which favour the deposition of calcium and phosphorus in a physiological solution.

Although, in the description, reference is made specifically to titanium osteointegrative interfaces, clearly the invention put forward is also applicable to interfaces made of tantalum and of alloys of titanium and of tantalum.

## Claims

1. A biomimetic osteointegrative interface comprising a substrate (3) of biocompatible material such as titanium, tantalum, or their alloys, **characterized in that** it comprises, on the surface (3bis) of the substrate (3), a first layer (4) having a first concentration of oxide of the biocompatible material, enriched with a second concentration of phosphorus and with a third concentration of calcium, the ratio between the concentration of calcium and the concentration of phosphorus being greater than two, the biomimetic osteointegrative interface (1) further comprising a second layer (5) having a first surface (10) in contact with the first layer (4), and a second surface (11), the second layer (5) having a fourth concentration of oxide enriched with a fifth concentration of calcium and a sixth concentration of phosphorus, the fourth concentration being less than the fifth and sixth concentrations and the ratio between the fifth concentration of calcium and the sixth concentration of phosphorus being no less than three.

2. A biomimetic osteointegrative interface according to Claim 1, **characterized in that** the second layer (5) has a gradient of the fifth calcium concentration and of the sixth phosphorus concentration which gradient increases from the first surface (10) towards the second surface (11) of the second layer (5).

3. A biomimetic osteointegrative interface according to Claim 2, **characterized in that** the second layer (5) is hydrated and has -OH hydroxyl chemical coupling groups suitable for forming a plurality of nucleation centres (12).

4. A method for the biomimetic treatment of an osteointegrative interface on a substrate of biocompatible metal such as titanium, tantalum, or their alloys, comprising the following series of steps:
a) performing a first ASD anodic deposition treatment of the osteointegrative interface (1) in a calcium glycerophosphate solution,
b) performing a second ASD anodic deposition treatment of the osteointegrative interface (1) in a calcium hydroxide solution, and
c) performing an immersion of the osteointegrative interface (1) in a potassium hydroxide solution.

5. A method -for the biomimetic treatment of an osteointegrative interface on a substrate of biocompatible metal such as titanium, tantalum, or their alloys, comprising the following series of steps:
a) performing a first ASD anodic deposition treatment of the osteointegrative interface (1) in a calcium glycerophosphate solution,
b) performing a second ASD anodic deposition treatment of the osteointegrative interface (1) in a calcium hydroxide solution, and
c) performing an immersion of the osteointegrative interface (1) in a sodium hydroxide solution.

6. A method for the biomimetic treatment of an osteointegrative interface according to Claim 4 or Claim 5, **characterized in that** the electrolytic solution containing calcium glycerophosphate has a concentration of 0.015 ± 0.005 M and a temperature T within a range where -1°C < T < +1°C.

7. A method for the biomimetic treatment of an osteointegrative interface according to any one of Claims 4, 5 and 6, **characterized in that** the first ASD deposition takes place with a constant current equal to a first predetermined value and with a voltage increasing freely to a first final value.

8. A method for the biomimetic treatment of an osteointegrative interface according to any one of Claims 4, 5, 6 and 7, **characterized in that** the electrolytic solution containing calcium hydroxide has a concentration of 0.1 ± 0.02 M and a temperature T within a range where 2°C < T < 8°C.

9. A method for the biomimetic treatment of an osteointegrative interface according to any one of Claims 4, 5, 6, 7 and 8, **characterized in that** the second ASD anodic deposition provides, for a first period of time, for a constant current equal to a second predetermined current value and for a voltage increasing freely to a second final value, and further provides, for a second period of time, for a current decreasing freely to a third value and for a constant voltage equal to a third predetermined voltage value.

10. A method for the biomimetic treatment of an osteointegrative interface according to any one of Claims 4, 6, 7 and 8, **characterized in that** the immersion in potassium hydroxide solution takes place for a period of at least t = 24 hours and a temperature T within a range where 59°C < T < 61°C.

11. A method for the biomimetic treatment of an osteointegrative interface according to Claim 7, **characterized in that** the first predetermined voltage value is equal to about 350 V.

12. A method - for the biomimetic treatment of an osteointegrative interface according to Claims 7 and 9, **characterized in that** the first and second predetermined current values are identical and are equal to about 70 A/m².

13. A method for the biomimetic treatment of an osteointegrative interface according to Claim 9, **characterized in that** the second and third final voltage values are identical and are equal to about 370 V.

14. A method for the biomimetic treatment of an osteointegrative interface according to Claim 9 or Claim 11, **characterized in that** the third predetermined current value is equal to half of the second predetermined current value and is equal to about 35 A/m².

## Patentansprüche

1. Biomimetische osteointegrative Grenzfläche, umfassend ein Substrat (3) aus biokompatiblem Material wie beispielsweise Titan, Tantal oder deren Legierungen, **dadurch gekennzeichnet, dass** es auf der Oberfläche (3bis) des Substrats (3) eine erste Schicht (4) umfasst, die eine erste Konzentration an Oxid des biokompatiblen Materials aufweist, angereichert mit einer zweiten Konzentration an Phosphor und mit einer dritten Konzentration an Calzium, wobei das Verhältnis zwischen der Konzentration an Calzium und der Konzentration an Phosphor größer als zwei ist, wobei die biomimetische osteointegrative Grenzfläche (1) femer eine zweite Schicht (5) mit einer ersten Oberfläche (10) in Kontakt mit der ersten Schicht (4) und eine zweite Oberfläche (11) aufweist, wobei die zweite Schicht (5) eine vierte Konzentration an Oxid, angereichert mit einer fünften Konzentration an Calzium und einer sechsten Konzentration an Phosphor aufweist, wobei die vierte Konzentration niedriger als die fünfte und die sechste Konzentration ist und das Verhältnis zwischen der fünften Konzentration an Calzium und der sechsten Konzentration an Phosphor nicht kleiner als drei ist.

2. Biomimetische osteointegrative Grenzfläche nach Anspruch 1, **dadurch gekennzeichnet, dass** die zweite Schicht (5) einen Gradienten der fünften Calzium-Konzentration und der sechsten Phosphor-Konzentration aufweist, der von der ersten Oberfläche (10) in Richtung der zweiten Oberfläche (11) der zweiten Schicht (5) hin ansteigt.

3. Biomimetische osteointegrative Grenzfläche nach Anspruch 2, **dadurch gekennzeichnet, dass** die zweite Schicht (5) hydratisiert ist und chemische -OH Hydroxyl-Kopplungsgruppen aufweist, die zur Bildung einer Vielzahl von Keimbildungszentren (12) geeignet sind.

4. Verfahren zur biomimetischen Behandlung einer osteointegrativen Grenzfläche auf einem Substrat aus biokompatiblem Metall wie beispielsweise Titan, Tantal oder deren Legierungen, welches die folgende Abfolge von Schritten umfasst:
a) Durchführen einer ersten ASD anodischen Ablagerungsbehandlung der osteointegrativen Grenzfläche (1) in einer Calziumgycerophosphat-Lösung,
b) Durchführen einer zweiten ASD anodischen Ablagerungsbehandlung der osteointegrativen Grenzfläche (1) in einer Calziumhydroxid-Lösung, und
c) Eintauchen der osteointegrativen Grenzfläche (1) in einer Kaliumhydroxid-Lösung.

5. Verfahren zur biomimetischen Behandlung einer osteointegrativen Grenzfläche auf einem Substrat aus biokompatiblem Metall wie beispielsweise Titan, Tantal oder deren Legierungen, welches die folgende Abfolge von Schritten umfasst:
a) Durchführen einer ersten ASD anodischen Ablagerungsbehandlung der osteointegrativen Grenzfläche (1) in einer Calziumgycerophosphat-Lösung,
b) Durchführen einer zweiten ASD anodischen Ablagerungsbehandlung der osteointegrativen Grenzfläche (1) in einer Calziumhydroxid-Lösung, und
c) Eintauchen der osteointegrativen Grenzfläche (1) in einer Natriumhydroxid-Lösung.

6. Verfahren zur biomimetischen Behandlung einer osteointegrativen Grenzfläche nach Anspruch 4 oder Anspruch 5, **dadurch gekennzeichnet, dass** die elektrolytische Lösung welche Calziumglycerophosphat enthält eine Konzentration von 0,015 ± 0,005 M und eine Temperatur T innerhalb eines Bereichs von -1°C < T < +1°C aufweist.

7. Verfahren zur biomimetischen Behandlung einer osteointegrativen Grenzfläche nach einem der Ansprüche 4, 5 und 6, **dadurch gekennzeichnet, dass** die erste ASD Ablagerung mit einem konstanten Strom der gleich einem ersten vorherbestimmten Wert ist und mit einer frei auf einen ersten Endwert ansteigenden Spannung erfolgt.

8. Verfahren zur biomimetischen Behandlung einer osteointegrativen Grenzfläche nach einem der Ansprüche 4, 5, 6 und 7, **dadurch gekennzeichnet, dass** die elektrolytische Lösung welche Calziumhydroxid enthält eine Konzentration von 0,1 ± 0,02 M und eine Temperatur T innerhalb eines Bereichs von 2°C < T < 8° C aufweist.

9. Verfahren zur biomimetischen Behandlung einer osteointegrativen Grenzfläche nach einem der Ansprüche 4, 5, 6, 7 und 8, **dadurch gekennzeichnet, dass** die zweite ASD anodische Ablagerung für einen ersten Zeitraum einen konstanten Strom, der gleich einem zweiten vorherbestimmten Stromwert ist und eine frei auf einen zweiten Endwert ansteigende Spannung vorsieht und dass sie femer für einen zweiten Zeitraum einen frei auf einen dritten Wert abfallenden Strom und eine konstante Spannung die gleich einem dritten vorherbestimmten Spannungswert ist, vorsieht.

10. Verfahren zur biomimetischen Behandlung einer osteointegrativen Grenzfläche nach einem der Ansprüche 4, 6, 7 und 8, **dadurch gekennzeichnet, dass** das Eintauchen in Kaliumhydroxid-Lösung für einen Zeitraum von wenigstens 24 Stunden und bei einer Temperatur innerhalb eines Bereichs von 59°C < T < 61° C erfolgt.

11. Verfahren zur biomimetischen Behandlung einer osteointegrativen Grenzfläche nach einem Anspruch 7, **dadurch gekennzeichnet, dass** der erste vorherbestimmte Spannungswert gleich etwa 350 V ist.

12. Verfahren zur biomimetischen Behandlung einer osteointegrativen Grenzfläche nach den Ansprüchen 7 und 9, **dadurch gekennzeichnet, dass** der erste und der zweite vorherbestimmte Stromendwert identisch sind, und dass sie gleich etwa 70 A/m² sind.

13. Verfahren zur biomimetischen Behandlung einer osteointegrativen Grenzfläche nach Anspruch 9, **dadurch gekennzeichnet, dass** der zweite und der dritte Spannungsendwert identisch sind, und dass sie gleich etwa 370 V sind.

14. Verfahren zur biomimetischen Behandlung einer osteointegrativen Grenzfläche nach Anspruch 9 oder Anspruch 11, **dadurch gekennzeichnet, dass** der dritte vorherbestimmte Stromwert halb so groß ist wie der zweite vorherbestimmte Stromwert, und dass er gleich etwa 35 A/m² ist.

## Revendications

1. Interface d'ostéointégration biomimétique comprenant un substrat (3) de matériau biocompatible tel que le titane, le tantale, ou leurs alliages, **caractérisé en ce qu'**il comprend, sur la surface (3 bis) du substrat (3), une première couche (4) ayant une première concentration d'oxyde du matériau biocompatible, enrichie avec une deuxième concentration de phosphore et avec une troisième concentration de calcium, le rapport entre la concentration de calcium et la concentration de phosphore étant supérieur à deux, l'interface d'ostéointégration biomimétique (1) comprenant en outre une deuxième couche (5) ayant une première surface (10) en contact avec la première couche (4), et une deuxième surface (11), la deuxième couche (5) ayant une quatrième concentration d'oxyde enrichie avec une cinquième concentration de calcium et une sixième concentration de phosphore, la quatrième concentration étant inférieure à la cinquième et à la sixième concentrations et le rapport entre la cinquième concentration de calcium et la sixième concentration de phosphore étant non inférieur à trois.

2. Interface d'ostéointégration biomimétique selon la revendication 1, **caractérisé en ce que** la deuxième couche (5) a un gradient de la cinquième concentration de calcium et de la sixième concentration de phosphore, lequel gradient augmente depuis la première surface (10) vers la deuxième surface (11) de la deuxième couche (5).

3. Interface d'ostéointégration biomimétique selon la revendication 2, **caractérisé en ce que** la deuxième couche (5) est hydratée et possède des groupes de couplage chimique hydroxyle -OH convenables pour former une pluralité de centres de nucléation (12).

4. Procédé pour le traitement biomimétique d'une interface d'ostéointégration sur un substrat de métal biocompatible tel que le titane, le tantale, ou leurs alliages, comprenant la série d'étapes suivantes :
a) exécution d'un premier traitement par dépôt anodique ASD de l'interface d'ostéointégration (1) dans une solution de glycérophosphate de calcium,
b) exécution d'un deuxième traitement par dépôt anodique ASD de l'interface d'ostéointégration (1) dans une solution d'hydroxyde de calcium, et
c) exécution d'une immersion de l'interface d'ostéointégration (1) dans une solution d'hydroxyde de potassium.

5. Procédé pour le traitement biomimétique d'une interface d'ostéointégration sur un substrat de métal biocompatible tel que le titane, le tantale, ou leurs alliages, comprenant la série d'étapes suivantes :
a) exécution d'un premier traitement par dépôt anodique ASD de l'interface d'ostéointégration (1) dans une solution de glycérophosphate de calcium,
b) exécution d'un deuxième traitement par dépôt anodique ASD de l'interface d'ostéointégration (1) dans une solution d'hydroxyde de calcium, et
c) exécution d'une immersion de l'interface d'ostéointégration (1) dans une solution d'hydroxyde de potassium.

6. Procédé pour le traitement biomimétique d'une interface d'ostéointégration selon la revendication 4 ou la revendication 5, **caractérisé en ce que** la solution électrolytique contenant le glycérophoephate de calcium a une concentration de 0,015 ± 0,005 M et une température T à l'intérieur d'une plage où -1°C < T < +1°C.

7. Procédé pour le traitement biomimétique d'une interface d'ostéointégration selon l'une quelconque des revendications 4, 5 et 6, **caractérisé en ce que** le premier dépôt ASD se déroule avec un courant constant égal à une première valeur prédéterminée et avec une tension qui s'élève librement jusqu'à une première valeur finale.

8. Procédé pour le traitement biomimétique d'une interface d'ostéointégration selon l'une quelconque des revendications 4, 5, 6 et 7, **caractérisé en ce que** la solution électrolytique contenant l'hydroxyde de calcium a une concentration de 0,1 ± 0,02 M et une température T à l'intérieur d'une plage où 2°C < T < 8°C.

9. Procédé pour le traitement biomimétique d'une interface d'ostéointégration selon l'une quelconque des revendications 4, 5, 6, 7 et 8, **caractérisé en ce que** le deuxième dépôt anodique ASD produit, pendant une première période de temps, un courant constant égal à une deuxième valeur de courant prédéterminée et une tension qui s'élève librement jusqu'à une deuxième valeur finale, et produit en outre, pendant une deuxième période de temps, un courant qui retombe librement jusqu'à une troisième valeur et une tension constante égale à une troisième valeur de tension prédéterminée.

10. Procédé pour le traitement biomimétique d'une interface d'ostéointégration selon l'une quelconque des revendications 4, 6, 7 et 8, **caractérisé en ce que** l'immersion dans la solution d'hydroxyde de potassium se déroule pendant une période d'au moins t = 24 heures et à une température T à l'intérieur d'une plage où 59°C < T < 61°C.

11. Procédé pour le traitement biomimétique d'une interface d'ostéointégration selon la revendication 7, **caractérisé en ce que** la première valeur de tension prédéterminée est égale à environ 350 V.

12. Procédé pour le traitement biomimétique d'une interface d'ostéointégration selon les revendications 7 et 9, **caractérisé en ce que** la première et la deuxième valeurs de courant prédéterminées sont identiques et sont égales à environ 70 A/m².

13. Procédé pour le traitement biomimétique d'une interface d'ostéointégration selon la revendication 9, **caractérisé en ce que** la deuxième et la troisième valeurs de tension finales sont identiques et sont égales à environ 370 V.

14. Procédé pour le traitement biomimétique d'une interface d'ostéointégration selon la revendication 9 ou la revendication 11, **caractérisé en ce que** la troisième valeur de courant prédéterminée est égale à la moitié de la deuxième valeur de courant prédéterminée et est égale à environ 35 A/m².
